# EUROPÄISCHE PATENTSCHRIFT

(11) **EP 2 679 752 B1**
(45) Veröffentlichungstag und Bekanntmachung des Hinweises auf die Patenterteilung: **27.03.2019**
(21) Anmeldenummer: 13173340.4
(22) Anmeldetag: 24.06.2013
(51) Int. Cl.: F04B 43/08, F04B 43/12, A61M 5/142, E05D 11/10

(54) **Medizinisches Gerät zur extrakorporalen Blutbehandlung mit einer Deckelvorrichtung zur schwenkbaren Halterung eines Deckels an dem medizinischen Gerät**
Medical device for extracorporeal blood treatment with a cover and hinge arrangement for the pivotable fixing of the cover to the medical device
Appareil médical pour le traitement sanguin extracorporel avec un couvercle pour le support pivotant du couvercle sur l'appareil médical

(30) Priorität: 27.06.2012 DE 102012105614
(43) Veröffentlichungstag der Anmeldung: 01.01.2014
(73) Patentinhaber: B. Braun Avitum AG, 34212 Melsungen (DE)
(72) Erfinder: Schäfer, Oliver, 36286 Neuenstein (DE)
(74) Vertreter: Winter, Brandl, Fürniss, Hübner, Röss, Kaiser, Polte - Partnerschaft mbB

(56) Entgegenhaltungen:
- EP-A1- 0 019 205
- GB-A- 191 307 534
- US-A- 663 571
- US-A- 5 928 177
- US-B2- 7 547 200

## Beschreibung

Die Erfindung betrifft ein medizinisches Gerät zur extrakorporalen Blutbehandlung mit einer Deckelvorrichtung, umfassend einen Deckel und ein zweites Bauteil, wobei das zweite Bauteil Teil des medizinischen Geräts ist und der Deckel schwenkbar an dem zweiten Bauteil angebracht und dazu ausgebildet ist, das zweite Bauteil in einer geschlossenen Lage wenigstens teilweise abzudecken, wobei der Deckel gegenüber dem zweiten Bauteil in wenigstens einer Lage verrastbar ist.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt. Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

Beispielsweise offenbart die Offenlegungsschrift DE 10 2007 020 573 A1 eine solche Schlauchrollenpumpe mit einem Stator, einem Rotor und einem Rotorantrieb, bei der zwischen den Rotor und die Schlauchrollenbahn des Stators ein Schlauch eingelegt wird. Durch die Rotation des Rotors und damit die Umlaufbewegung von Schlauchrollen wird der Schlauch jeweils gegen die Schlauchrollenbahn des Stators gedrückt, wodurch Flüssigkeit durch den Schlauch gepumpt wird.

Auch die Patentschrift US 7,547,200 B2 offenbart eine solche peristaltische Pumpe mit einem Rotor und Rollen, welche einen eingelegten Schlauch gegen eine halbkreisförmige Schlauchrollenbahn drücken. Dabei hat die Rollenbahn an einem Ende eine abgeschrägte Kante, um den Schlauch aufzunehmen, der an einem auswechselbaren Einsatz angebracht ist.

Patentschrift US 5,928,177 A offenbart eine Deckelvorrichtung für eine Dialysemaschine, wobei ein Deckel der Deckelvorrichtung während eines geschlossenen Zustandes eine peristaltische Pumpe abdeckt und gleichzeitig einen integralen Teil der peristaltischen Pumpe darstellt.

In der Patentschrift GB 191307534 A ist beispielsweise eine mögliche Ausführungsform eines arretierbaren Schwenkgelenks offenbart. Dabei wirkt ein durch eine Druckfeder vorgespannter Kolben derart auf einen Teil des Schwenkgelenks ein, dass das Schwenkgelenk nur unter Überwindung der Kolbenvorspannung um eine bezüglich des Schwenkgelenks raumfeste Schwenkachse schwenkbar ist.

Die Patentschrift US 663,571 A offenbart ein Gelenk für Schwingtüren, bei welchem die Drehachse beim Öffnen und Schließen verschoben wird.

Die in der Medizintechnik bei solchen Pumpen verwendeten Überleitsysteme werden üblicherweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird. Daher werden zugehörige Deckel der Schlauchrollenpumpe mindestens vier Mal pro Behandlung betätigt, um das Überleitsystem an dem medizinischen Gerät aufzurüsten und wieder abzurüsten.

Üblicherweise weisen die verwendeten Deckel wenigstens ein Scharnier auf, um welches der Deckel zwischen einer geöffneten und einer geschlossenen Stellung schwenkbar ist. Um die Handhabung des Deckels zu unterstützen, ist es ferner bekannt, verschiedene definierte Halte- bzw. Rastpositionen vorzusehen. Beispielsweise kann die geschlossene Stellung des Deckels durch eine Position definiert sein, in welcher der Deckel mittels Magnetkraft am Pumpengehäuse gehalten wird. Dazu kann ein im Deckel oder Pumpengehäuse verbauter Magnet mit einem im Deckel oder Pumpengehäuse verbauten ferromagnetischen Material zusammenwirken.

Eine bekannte technische Lösung für eine Verrastungsfunktion in einem definierten Öffnungswinkel besteht aus feststehenden Drehpunkten in Verbindung mit einem Halteelement, das beispielsweise durch einen Druckfederbolzen gebildet werden kann, der eine definierte Kraft auf eine ebene Fläche des Deckels aufbringt. Allerdings kann die Anordnung des Halteelements bei derartigen Systemen nachteilig sein, insbesondere wenn ein federbelasteter Druckbolzen auf einer mittig angeordneten Funktionsfläche des typischerweise transparenten Kunststoffdeckels angreift. Durch eine solche Anordnung bringt das System hohe Belastungen in den Deckel ein, was an dem Krafteintrittspunkt zu negativen Verschleißerscheinungen führen kann.

Aufgabe der Erfindung ist es daher, ein medizinisches Gerät zur extrakorporalen Blutbehandlung mit einer Deckelvorrichtung bereitzustellen, mit welcher ein Deckel gegenüber einem Bauteil des medizinischen Geräts in wenigstens einer Lage verrastbar ist, wobei die Deckelvorrichtung einfach zu montieren ist und im Betrieb zu möglichst geringen Verschleißerscheinungen führt.

Ferner ist es Aufgabe der Erfindung, ein medizinisches Gerät zur extrakorporalen Blutbehandlung mit einer solchen Deckelvorrichtung bereitzustellen, wobei sich die Deckelvorrichtung insbesondere zur Abdeckung einer Schlauchrollenpumpe eines medizinischen Geräts zur extrakorporalen Blutbehandlung eignen soll.

Erfindungsgemäß wird diese Aufgabe durch ein medizinisches Gerät mit einer Deckelvorrichtung gemäß dem unabhängigen Anspruch 1 gelöst. Vorteilhafte Weiterbildungen des medizinischen Geräts und der Deckelvorrichtung ergeben sich aus den Unteransprüchen.
Das erfindungsgemäße medizinische Gerät zur extrakorporalen Blutbehandlung verfügt über eine Deckelvorrichtung mit einem Deckel, der über zumindest ein Schwenkgelenk an dem medizinischen Gerät angelenkt ist. Dabei weist das Schwenkgelenk einen, vorzugsweise am Deckel angeordneten, ersten Gelenkabschnitt und einen, vorzugsweise mit dem medizinischen Gerät verbindbaren, zweiten Gelenkabschnitt auf.

Die Gelenkabschnitte sind zumindest zwischen einer ersten Lage, in der der Deckel geöffnet ist, und einer zweiten Lage, in der der Deckel zumindest einen Teil des medizinischen Geräts abdeckt, zueinander um eine Schwenkachse verschwenkbar und in zumindest einer Schwenklage lösbar arretierbar oder verrastbar sind.

Die Deckelvorrichtung weist ferner zumindest ein Kontaktflächenpaar auf, das senkrecht zur Schwenkachse federelastisch gegeneinander vorgespannt ist, wobei zumindest eine der Kontaktflächen eine Abroll- und Rastgeometrie aufweist, die so ausgebildet ist, dass der Deckel in der zweiten Lage verrastet und die Schwenkbeweglichkeit des Schwenkgelenks nur unter Überwindung der federelastischen Vorspannkraft wieder freigegeben wird.

Durch die federelastische Vorspannung wird erreicht, dass die Kontaktflächen der Deckelvorrichtung während der Relativdrehung der Gelenkabschnitte stets gegeneinander drücken bzw. aneinander abrollen bzw. abwälzen. Durch die Abroll- und Rastgeometrie ändert sich der Drehpunkt des Schwenkgelenks bezüglich des medizinischen Geräts bzw. bezüglich des Deckels. In der zweiten bzw. verrasteten Lage kommt die Rastgeometrie, z.B. eine Abflachung, in Anlage mit der Kontaktfläche am anderen Bauteil, wodurch sich der relative Abstand der Schwenkachse unter Zuhilfenahme der Federvorspannung verkürzt. Um das Schwenkgelenk von der verrasteten Lage wieder in eine nicht verrastete Lage bzw. Schwenklage zu bringen, d.h. um die Abrollgeometrie, z.B. eine Kreisbogenfläche, in Anlage mit der Kontaktfläche am anderen Bauteil zu bringen und somit den relativen Abstand der Schwenkachse wieder zu vergrößern, muss die an anliegende federelastische Vorspannung überwunden werden. Wird diese vorbestimmte Kraft nicht aufgebracht, verbleibt der Deckel selbstständig in der verrasteten Lage.

Erfindungsgemäß kann das medizinische Gerät einen Gehäuseabschnitt mit wenigstens einem Haltesystem aufweisen, welches einen Deckelhalter als zweiten Gelenkabschnitt umfasst, der federbelastet gegenüber dem medizinischen Gerät bewegbar ist. Der Deckel ist schwenkbar um eine Drehachse an diesem Deckelhalter gelagert, wobei durch den federbelasteten Deckelhalter eine Zugkraft auf den Deckel aufbringbar ist. Die Kontaktfläche mit der Abroll- und Rastgeometrie kann an dem am Deckelangeordneten ersten Gelenkabschnitt ausgebildet sein. Diese Abroll- und Rastgeometrie kann eine Rastfläche bzw. Abflachung und wenigstens eine konvexe Abrollkurve umfassen, wobei die Abrollkurve an einer Seite an die Rastfläche angrenzt. In der verrasteten Lage des Deckels liegt die Rastfläche dann an der anderen Kontaktfläche des Kontaktflächepaars an, während die Abrollkurve beim Schwenken des Deckels aus der verrasteten Lage entlang der anderen Kontaktfläche des Kontaktflächepaars abgleitet.

Die Rastfläche ist gegenüber der Abrollkurve so ausgebildet, dass der Deckel nach Durchlaufen der Abrollkurve in die Rastfläche einrastet. Dabei kann die Rastfläche selbst unterschiedlich ausgeformt sein. Beispielsweise handelt es sich um eine plane Fläche, oder die Rastfläche weist eine wellige oder gezackte Oberfläche auf. Insbesondere eine konkave Rastfläche hat sich hierbei als vorteilhaft erwiesen.

Bei der verrasteten Lage des Deckels kann es sich um eine geöffnete oder eine geschlossene Lage des Deckels in Bezug zum medizinischen Gerät handeln. Beispielsweise ist der Deckel in der geöffneten Lage verrastbar, um einem Bediener Zugriff auf das medizinischen Gerät zu gewähren, ohne dass der Deckel von selbst zurück in die geschlossene Lage schwenken kann, da dies gegebenenfalls Handlungen des Bedieners am zweiten Bauteil stören könnte. In dieser Ausführungsform deckt der Deckel das medizinischen Gerät somit in der verrasteten Lage nicht ab. Es sind jedoch auch Anwendungen denkbar, bei denen ein Deckel in einer geschlossenen Lage verrastet werden soll, damit sich dieser nicht unbeabsichtigt öffnet.

Die Verrastung wird im Wesentlichen durch die Zugkraft bewirkt, welche den Deckelhalter und damit auch die Abroll- und Rastgeometrie vorzugsweise permanent gegen das zweite Bauteil zieht. In der verrasteten Lage liegt die Rastfläche der Abroll- und Rastgeometrie an dem zweiten Bauteil an, und der Deckel kann nur durch eine definierte Kraft aus dieser verrasteten Lage geschwenkt werden, denn die Federkraft muss überwunden werden, um die Abroll- und Rastgeometrie so zu drehen, dass nun eine konvexe Abrollkurve an der anderen Kontaktfläche des Kontaktflächenpaars abgleiten kann. Je nach Ausformung der Abroll- und Rastgeometrie kann auch erreicht werden, dass der Deckel auch in dieser zweiten Lage gesichert ist.

In einem Ausführungsbeispiel der Erfindung umfasst das Haltesystem eine Gleitlagerbuchse, innerhalb welcher der Deckelhalter axial beweglich gelagert ist, wobei die Gleitlagerbuchse in einem Gehäuseabschnitt des medizinischen Geräts fixiert ist. Das Haltesystem kann so über die Gleitlagerbuchse beispielsweise einfach in eine Bohrung im Gehäuseabschnitt eingepresst werden. Dabei ist der Deckelhalter in einer Ausführungsform der Erfindung zylinderförmig, was den Vorteil mit sich bringt, dass er auch in Umfangsrichtung innerhalb der Gleitlagerbuchse beweglich ist. Dies erleichtert insbesondere die Montage des Haltesystems, da es nicht an dem Gehäuseabschnitt ausgerichtet werden muss. Vielmehr kann die Gleitlagerbuchse mit dem darin befindlichen Deckelhalter einfach in einer Bohrung im Gehäuseabschnitt montiert und der Deckelhalter dann innerhalb der montierten Gleitlagerbuchse so gedreht werden, dass der Deckel daran angebracht werden kann. Um die Verbindung des Deckels mit dem Deckelhalter zu vereinfachen, kann es jedoch vorgesehen sein, den Deckelhalter im Bereich der Lagerung des Deckels plattenförmig auszuformen. So kann er im Deckel beispielsweise in eine entsprechende Bohrung mit einem rechteckigen Querschnitt eingeführt werden, wodurch sich eine Ausrichtung des Deckelhalters in Umfangsrichtung im Deckel realisieren lässt. Durch diese definierte Ausrichtung wird beispielsweise die Einbringung eines Scharnierstifts durch eine Bohrung im Deckelhalter erleichtert.

In einer Ausführungsform der Erfindung ragt der Deckelhalter mit zwei gegenüber liegenden Enden aus der Gleitlagerbuchse heraus, wobei an einem Ende eine Lagerung für den Deckel ausgebildet ist, während der Deckelhalter am anderen Ende einen Anschlag aufweist, und das Haltesystem ferner eine Druckfeder umfasst, welche den Deckelhalter umgibt und zwischen dem Anschlag und der Gleitlagerbuchse gespannt ist. Durch diesen Aufbau des Haltesystems kann die Zugkraft auf den Deckel realisiert werden, denn die gespannte Druckfeder drückt den Anschlag von der Gleitlagerbuchse weg, wodurch auf der anderen Seite die Lagerung des Deckelhalters und damit der daran angebrachte Deckel permanent in Richtung der Gleitlagerbuchse gezogen werden. Die Zugkraft auf den Deckelhalter kann jedoch auch durch andere federbelastete Konstruktionen erzeugt werden.

In einer Ausführung kann die andere Kontaktfläche des Kontaktflächenpaars durch die Außenfläche des Gehäuseabschnitts des medizinischen Geräts gebildet werden. Dann liegt die Rastfläche in der verrasteten Lage des Deckels an der Außenfläche an und die wenigstens eine Abrollkurve gleitet beim Schwenken des Deckels aus der verrasteten Lage entlang der Außenfläche ab.

Vorzugsweise sind jedoch die Abmessungen der Abroll- und Rastgeometrie und des Haltesystems so gewählt, dass die Rastfläche in der verrasteten Lage des Deckels an der Gleitlagerbuchse anliegt, und die wenigstens eine Abrollkurve beim Schwenken des Deckels aus der verrasteten Lage entlang der Gleitlagerbuchse abgleitet. Das heißt, die Abroll- und Rastgeometrie am einen Gelenkabschnitt einerseits und die Stirnfläche der Gleitlagerbuchse anderseits bilden ein Paar von federelastisch gegeneinander vorgespannten Kontaktflächen. Dies hat den Vorteil, dass es auf dem Material des Gehäuseabschnitts zu keinen Verschleißerscheinungen kommt, denn Druck und Reibung durch den Deckel wirken nur auf das Haltesystem und nicht auf das Material des Gehäuseabschnitts. Dazu kann das Material beispielsweise der Gleitlagerbuchse so gewählt werden, dass es dieser Beanspruchung ohne Beeinträchtigung standhält. Insbesondere kann die Gleitlagerbuchse aus einem widerstandsfähigerem Material ausgeformt sein als der Gehäuseabschnitt selbst, was das Design von Bauteilen erleichtern kann, für welche das erfindungsgemäße Deckelvorrichtung verwendet wird.

Es kann jedoch auch vorgesehen sein, dass die Rastfläche in der verrasteten Lage des Deckels wenigstens teilweise an dem Gehäuse anliegt, und die wenigstens eine Abrollkurve beim Schwenken des Deckels aus der verrasteten Lage wenigstens teilweise entlang des Gehäuses abgleitet.

In einer weiteren Ausführungsform der Erfindung weist die Abroll- und Rastgeometrie zwei konvexe Abrollkurven auf, welche auf gegenüber liegenden Seiten an die Rastfläche angrenzen. Hierdurch kann in der verrasteten Lage ein Überlastschutz erreicht werden, denn auch beim Schwenken des Deckels über die verrastete Lage hinaus rollt die Abroll- und Rastgeometrie kontrolliert an der anderen Kontaktfläche ab, ohne dass es zu Beschädigungen kommt. Ein derartiges Schwenken wird zwar durch die Federkraft erschwert, ist jedoch nicht unmöglich, so dass beispielsweise ein unbeabsichtigtes Schwenken des Deckels nicht zu Brüchen oder Verbiegungen von Bauteilen führt.

Vorzugsweise ist zwischen der wenigstens einen Abrollkurve und der Rastfläche eine Kante ausgebildet. Die Rastfläche geht somit nicht gleichmäßig in eine Abrollkurve über, sondern beim Schwenken aus der verrasteten Lage heraus muss erst eine Kante überwunden werden.

Der Deckel ist beispielsweise über eine Scharnierverbindung an dem Deckelhalter gelagert. Dabei kann im Deckel eine Scharnierbohrung vorgesehen sein, durch welche die Drehachse verläuft. Wenigstens eine zweite Bohrung verläuft dann quer durch diese Scharnierbohrung, wobei der Deckelhalter so in die zweite Bohrung eingefügt ist, dass eine Stifthalterung des Deckelhalters innerhalb der Scharnierbohrung liegt und von einem Scharnierstift durchdrungen ist, der in die Scharnierbohrung eingeschoben ist. Der Scharnierstift verbindet den Deckel und den Deckelhalter über eine Spielpassung, was eine leichte Montage ermöglicht und auch den Service an der Deckelvorrichtung sehr einfach gestaltet.

Dabei kann der Scharnierstift axial innerhalb der Scharnierbohrung fixiert sein, um ihn gegen Herausfallen zu sichern. Dies kann beispielsweise durch eine umlaufende Nut am Scharnierstift realisiert werden, in welche in der Endlage des Scharnierstifts innerhalb der zugehörigen Scharnierbohrung eine Erhebung im Deckel eingreift. Es können jedoch auch andere Arten von Verrastungsgeometrien vorgesehen sein, wobei die permanent anliegende Federkraft, welche quer zum Scharnierstift wirkt, die axiale Fixierung des Scharnierstifts unterstützt.

Der Deckel kann dabei mit einem oder mehreren Haltesystemen am medizinischen Gerät angebracht sein. Beispielsweise können wenigstens zwei Haltesysteme vorgesehen sein, welche einen Deckel an zwei Seiten schwenkbar lagern.

Die erfindungsgemäße Deckelvorrichtung eignet sich zur Abdeckung verschiedener Bauteile eines medizinischen Geräts zur extrakorporalen Blutbehandlung, wobei ein Deckel gegenüber einem Bauteil in einer bestimmten Lage gehalten werden soll. Insbesondere an Komponenten, die eine wiederholte manuelle Betätigung erfordern, zwischen den Betätigungsphasen aber gegen Einflüsse von außen geschützt sein sollen, hat sich die Deckelvorrichtung als vorteilhaft erwiesen. Dies ist beispielsweise bei

Schlauchrollenpumpen an Dialysegeräten der Fall, da dort Einweg-Schlauchsegmente wiederholt ein- und ausgebaut werden müssen.

In einer Ausführungsform der Erfindung umfasst ein medizinisches Gerät zur extrakorporalen Blutbehandlung daher wenigstens eine Schlauchrollenpumpe mit einem Pumpengehäuse, das eine gebogene Lauffläche und einen innerhalb der Lauffläche drehenden Rotor aufweist, wobei ein Schlauchsegment eines extrakorporalen Blutkreislaufs zwischen die Lauffläche und den Rotor einbringbar ist. Die Schlauchrollenpumpe umfasst dabei eine erfindungsgemäße Deckelvorrichtung, wobei das Pumpengehäuse in der geschlossenen Lage wenigstens teilweise durch den Deckel abdeckbar ist.

Die Verrastung kann dann beispielsweise dazu genutzt werden, den Deckel in einer geöffneten Lage zu halten, so dass ein Bediener Zugriff auf das Pumpengehäuse hat und den Rotor und/oder ein Schlauchsegment aufrüsten bzw. abrüsten kann, ohne dass der Deckel unbeabsichtigt zurück in seine geschlossene Lage schwenkt. Hierdurch lassen sich insbesondere die Anforderungen an eine Ein-Hand-Bedienung zum Einlegen und Entnehmen des Überleitsystems erfüllen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Darstellung einer Aufsicht auf eine Schlauchrollenpumpe;
- Fig. 3: eine schematische Seitenansicht auf eine Schlauchrollenpumpe mit geöffnetem Deckel;
- Fig. 4: eine schematische Seitenansicht auf eine Schlauchrollenpumpe mit geschlossenem Deckel;
- Fig. 5a: eine vergrößerte Ansicht eines Haltesystems mit geöffnetem Deckel;
- Fig. 5b: ein Haltesystem nach Fig. 4b beim Schließvorgang;
- Fig. 5c: ein Haltesystem nach Fig. 4b mit geschlossenem Deckel;
- Fig. 6: ein Haltesystem bei der Montage am Pumpengehäuse; und
- Fig. 7: eine Teilansicht eines Haltesystems mit montiertem Deckel.

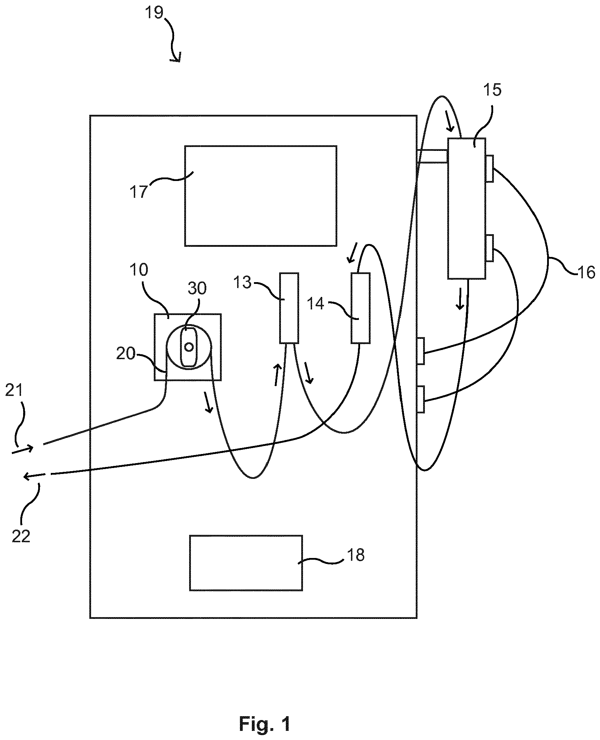

Fig. 1 zeigt eine schematische Darstellung der wesentlichen Grundkomponenten eines medizinischen Geräts 19 zur extrakorporalen Blutbehandlung mit einer Blutpumpe, wobei es sich bei der Blutpumpe insbesondere um eine Schlauchrollenpumpe handelt. Die Schlauchrollenpumpe weist dabei ein Pumpengehäuse 10 auf, das typischerweise an der Frontseite des Dialysegeräts 19 angebracht ist.

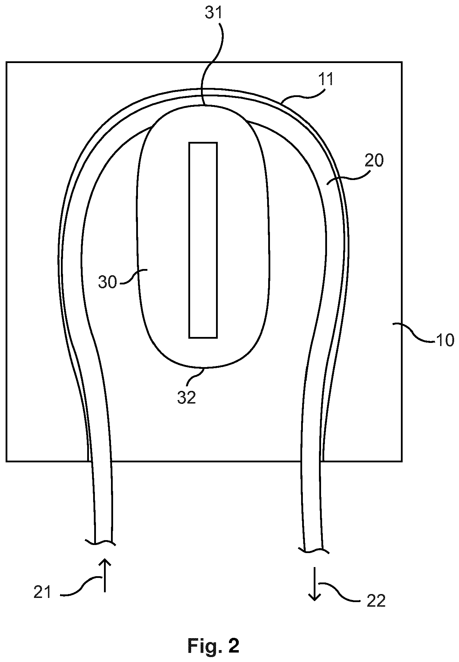

Dieser Schlauchrollenpumpe wird arterielles Blut 21 eines Patienten zugeführt und durch den extrakorporalen Blutkreislauf gefördert. Anschließend wird das Blut als venöses Blut 22 wieder zum Patienten zurückgeführt. Dabei wird das Blut mittels der Pumpe durch ein Überleitsystem gefördert, das an mehrere Komponenten des Dialysegeräts angeschlossen ist, wobei ein Schlauchsegment 20 des Überleitsystems in die Schlauchrollenpumpe eingelegt ist und ein Rotor 30 das Blut peristaltisch durch dieses Schlauchsegment 20 fördert, wie es einer vergrößerten Ansicht der Fig. 2 zu entnehmen ist.

Nach Durchlaufen der Blutpumpe gelangt das Blut zum Dialysator 15, nachdem es vorzugsweise zuvor einen arteriellen Luftfänger 13 durchlaufen hat. Im Dialysator 15 wird das Blut durch Stoffaustausch mit einem Dialysat 16 gereinigt, welches dem Dialysator 15 zu- und abgeführt wird. Nach Durchlaufen des Dialysators 15 gelangt das Blut zu einem venösen Luftfänger 14 und wird dann dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 17 erfolgen, die vorzugsweise als Touchscreen ausgebildet ist. Ferner weist das Dialysegerät 19 eine Steuer-/Regeleinheit 18 auf.

Fig. 2 stellt eine schematische Darstellung einer Aufsicht auf die Schlauchrollenpumpe mit eingelegtem Schlauchsegment 20 und Rotor 30 dar. Die Schlauchrollenpumpe weist dabei das Pumpengehäuse 10 auf, und die erfindungsgemäße Deckelvorrichtung eignet sich insbesondere zur schwenkbaren Anbringung eines Deckels an diesem Pumpengehäuse 10, um die Schlauchrollenpumpe während der Therapie abzudecken, dem Bediener jedoch bei Bedarf auch den Zugriff auf das Überleitungssystem 20 zu ermöglichen.

In dem Pumpengehäuse 10 ist durch eine Vertiefung im Pumpengehäuse 10 eine kurvenförmige Lauffläche 11 ausgebildet, in welche das Schlauchsegment 20 schlaufenförmig eingelegt werden kann, so dass seine beiden Schlauchenden unten aus dem Pumpengehäuse 10 herausragen. Dabei kann die Vertiefung mit einer Seitenfläche in dem Pumpengehäuse 10 ausgebildet sein, die im Wesentlichen gleichmäßig senkrecht zur Frontseite des Geräts verläuft, oder die Lauffläche 11 ist ungleichmäßig durch eine Seitenfläche der Vertiefung ausgeformt, die konkav oder sogar in sich verdreht ausgeformt ist.

Innerhalb der Lauffläche 11 ist ein Rotor 30 angebracht, der beispielsweise einen elliptischen Umfang hat, so dass er das Schlauchsegment 20 bei der Rotation an seinen Hauptscheiteln 31, 32 leicht zusammendrücken kann. Durch die Drehung des Rotors 30 beispielsweise im Uhrzeigersinn bewegt sich der Bereich eines zusammengedrückten Schlauchsegments ebenfalls im Uhrzeigersinn, bis sich der zugehörige Hauptscheitel wieder vom Schlauchsegment löst. In der Zeit hat der gegenüber liegende Hauptscheitel jedoch bereits wieder Kontakt zu dem Schlauchsegment 20 aufgenommen, so dass Blut jeweils in dem Bereich des Schlauchsegments 20, vor dem es von dem Rotor 30 zusammendrückt wird, peristaltisch vom Pumpeneingang zum Pumpenausgang gefördert wird. An den beiden Enden des Schlauchsegments 20 können dafür Konnektoren ausgebildet oder angebracht sein, welche eine Verbindung zu weiteren zu- und abführenden Leitungen herstellen.

Ja nach Ausrichtung des Pumpengehäuses 10 an dem medizinischen Gerät 19 ist der Deckel in Fig. 2 oben, unten oder seitlich gelagert, so dass er nach oben, unten oder zur Seite hin schwenkbar ist.

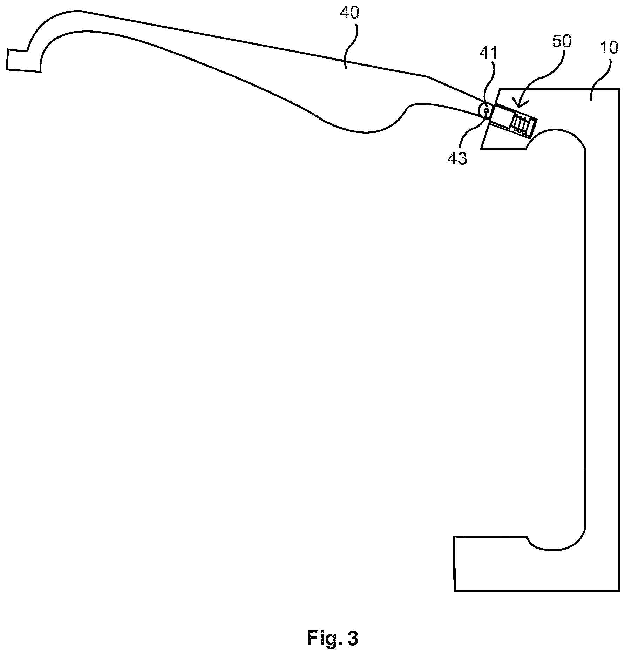

Fig. 3 zeigt eine schematische Seitenansicht auf die Schlauchrollenpumpe, wobei dies in Fig. 2 einer Sicht von unten entspricht.

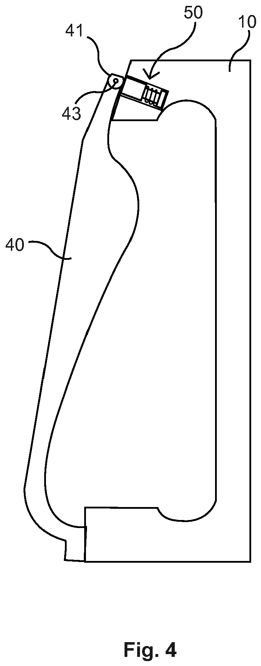

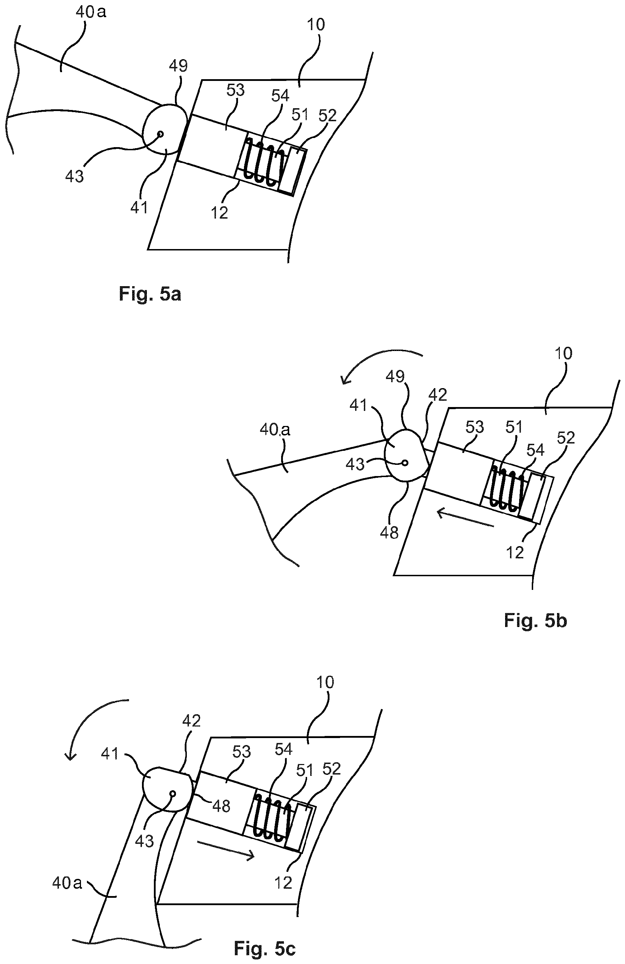

Der Deckel 40 deckt das Pumpengehäuse 10 mit seiner Lauffläche 11 und dem Rotor 30 vorzugsweise vollständig ab und ist schwenkbar im (in Fig. 3) oberen Bereich des Gehäuses 10 gelagert. Dazu ist ein Haltesystem 50 vorgesehen, welches in eine Bohrung 12 (siehe Fig. 5a bis 5c) innerhalb des Pumpengehäuses 10 eingebracht ist. Der Deckel 40 ist bezüglich dieses Haltesystems 50 drehbar über eine Drehachse 43 gelagert, und Fig. 3 zeigt den Deckel 40 in einer geöffneten Lage. Fig. 4 dagegen zeigt den Deckel 40 in der geschlossenen Lage.

Die definierte geöffnete Lage der Fig. 3 wird durch ein Zusammenspiel der Geometrie des Deckels 40 und des gefederten Haltesystems 50 des Pumpengehäuses 10 erreicht. Das Gleiche gilt für die definierte geschlossene Lage der Fig. 4. Um dies näher zu erläutern, sind in den Figuren 5a bis 5c vergrößerte Ansichten dieser beiden Bereiche gezeigt. Fig. 5a zeigt den Deckel 40 erneut in der geöffneten Lage der Fig. 3. Hierbei ist ersichtlich, dass das Haltesystem 50 aus einem Deckelhalter 51, einer Gleitlagerbuchse 53, einem Anschlag 52 und einer Feder 54 besteht. Der Deckelhalter 51 ist im Wesentlichen zylinderförmig ausgeformt und weist an dem Ende, das sich innerhalb des Pumpengehäuses 10 befindet, einen plattenförmigen Anschlag 52 auf. Der Anschlag 52 kann jedoch auch auf andere Art ausgebildet werden, wie etwa durch abstehende Stifte, oder ein Ende der Feder 54 ist fest an dem Deckelhalter 51 fixiert. An dem gegenüber liegenden Ende des Deckelhalters 51 ist eine Stifthalterung in Form einer Bohrung vorgesehen, an welcher der Deckel 40 mittels eines Scharnierstifts anbringbar ist. Innerhalb dieser Bohrung befindet sich dann die Drehachse 43 des Deckels 40.

Der Deckelhalter 51 ist beweglich innerhalb einer Gleitlagerbuchse 53 geführt, welche in Form eines Hohlzylinders ausgebildet sein kann. Dabei ist die Gleitlagerbuchse 53 fest im Pumpengehäuse 10 verankert, was beispielsweise über eine Presspassung erfolgen kann. Alternativ oder ergänzend kann eine Verankerung der Gleitlagerbuchse 53 auch durch andere Verbindungsarten wie beispielsweise Klebe-, Stift- und/oder Schraubverbindungen erfolgen. Der zylinderförmige Deckelhalter 51 kann sich axial innerhalb der fest stehenden Buchse 53 bewegen. Zwischen der Gleitlagerbuchse 53 und dem Anschlag 52 befindet sich eine gespannte Druckfeder 54, welche den Deckelhalter 51 umgibt. Die Feder 54 wird je nach Richtung der Bewegung des Deckelhalters 51 innerhalb der Gleitlagerbuchse 53 weiter gestaucht oder etwas entspannt, wobei die Federkraft den Anschlag 52 von der Gleitlagerbuchse 53 wegdrückt und somit den Deckelhalter 51 in die Gleitlagerbuchse 53 hineinzieht. Vorzugsweise ist die Druckfeder 54 dabei so zwischen der Gleitlagerbuchse 53 und dem Anschlag 52 eingespannt, dass daraus eine permanente Zugkraft resultiert.

Der Pumpendeckel 40 weist neben einem plattenförmigen Deckelbereich, mit welchem das Pumpengehäuse 10 abdeckbar ist, im Bereich der Drehachse 43 einen Gelenkabschnitt 40a auf. Der Gelenkabschnitt 40a des Deckels 40 einerseits und der Deckelhalter 51 anderseits bilden ein Schwenkgelenk zum Verschwenken des Deckels 40 bezüglich des Pumpengehäuse 10. Der Gelenkabschnitt 40a des Deckels 40 ist mit einer Abroll- und Rastgeometrie 41 versehen. Diese Abroll- und Rastgeometrie 41 ist im Wesentlichen zylinderförmig ausgeformt, wobei die Drehachse 43 des Deckels 40 jedoch exzentrisch durch diese Abroll- und Rastgeometrie 41 verlaufen kann. Abweichend von einer rein zylindrischen Form weist die Abroll- und Rastgeometrie 41 für eine Rastfunktion wenigstens eine Rastfläche 42 auf. Diese kann eben und plan ausgeführt sein, oder die Rastfläche 42 weist eine wellige oder gezackte Oberfläche auf. Insbesondere kann eine konkave Rastfläche vorgesehen sein.

In der geöffneten Lage des Deckels 40 ist die Abroll- und Rastgeometrie 41 so ausgerichtet, dass die Rastfläche 42 an der Gleitlagerbuchse 53 anliegt. Durch die Federkraft der Feder 54 wird der Anschlag 52 von der Gleitlagerbuchse 53 weggedrückt, so dass der Deckelhalter 51 in die Gleitlagerbuchse 53 hineingezogen wird. Hierdurch wird eine Zugkraft auf den Deckel 40 ausgeübt, welche die Rastfläche 42 an die Gleitlagerbuchse 53 und/oder das Gehäuse 10 heranzieht. Hierdurch ist der Deckel 40 in dieser Stellung verrastet und kann nicht durch die Schwerkraft nach unten schwenken, wozu die Federkraft entsprechend gewählt ist.

Wird jedoch von einem Bediener gezielt Druck nach unten (in Fig. 3) auf den Deckel 40 ausgeübt, dreht sich die Abroll- und Rastgeometrie 41 gegen den Uhrzeigersinn um die Drehachse 43. Um diese Drehung vollziehen zu können, wird der Deckelhalter 51 leicht gegen die Federkraft aus der Gleitlagerbuchse 53 herausgezogen, denn die Kante der Rastfläche 42 muss überwunden werden. Dieser Vorgang ist in Fig. 5b gezeigt, wobei das Schwenken des Deckels 40 mit einem gebogenen Pfeil gekennzeichnet ist, während die Bewegung des Deckelhalters 51 mit einem geraden Pfeil gekennzeichnet ist. Hierdurch lässt sich die Verrastung des Deckels 40 in der definierten geöffneten Lage aufheben, und die Abroll- und Rastgeometrie 41 weist eine erste konvexe Abrollkurve 48 auf, entlang derer die Abroll- und Rastgeometrie 41 nun an der Gleitlagerbuchse 53 abgleitet. Die Federkraft kann dabei so gewählt sein, dass die Ansprüche an die Betätigungskräfte und die Haltekraft für eine Rastfunktion erfüllt sind.

Ferner kann die Federkraft so gewählt sein, das auch die Ansprüche an ein selbsttätiges aktives Schließen des Deckels 40 und das Halten in der geschlossenen Lage erfüllt sind. Diese geschlossene Lage ist in Fig. 5c gezeigt, wobei der Deckelhalter 51 durch die Federkraft wieder leicht in die Gleitlagerbuchse 53 hineingezogen werden konnte, und nun die konvexe Abrollkurve 48 an der Gleitlagerbuchse 53 anliegt. Hierbei ist die konvexe Abrollkurve 48 so definiert und ausgeformt, dass der Deckel 40 in dieser Position gehalten wird. Geöffnet werden kann er daher erst durch einen erheblichen Druck von unten gegen den Deckel 40.

Die Figuren 5a und 5b zeigen an der Abroll- und Rastgeometrie 41 ferner eine zweite konvexe Abrollkurve 49, die mit einer zweiten Kante an der anderen Seite der Rastfläche 42 ausgeformt ist. Wird der Deckel 40 aus der verrasteten Lage der Fig. 5a nach oben gedrückt, muss dazu zwar auch gegen die Federkraft die Kante zwischen Rastfläche 42 und der zweiten Abrollkurve 49 überwunden werden, aber der Deckel 40 kann theoretisch weiter nach oben geschwenkt werden. Hierdurch ist ein Überlastschutz gegeben.

Somit bildet die Abroll- und Rastgeometrie 41, d.h. die Rastfläche 42 und die Abrollkurven 48 und 49, eine erste Kontaktfläche und die Stirnfläche der Gleitlagerbuchse 53 eine zweite Kontaktfläche des erfindungsgemäßen Kontaktflächenpaars, die durch Federkraft aneinander gedrückt werden.

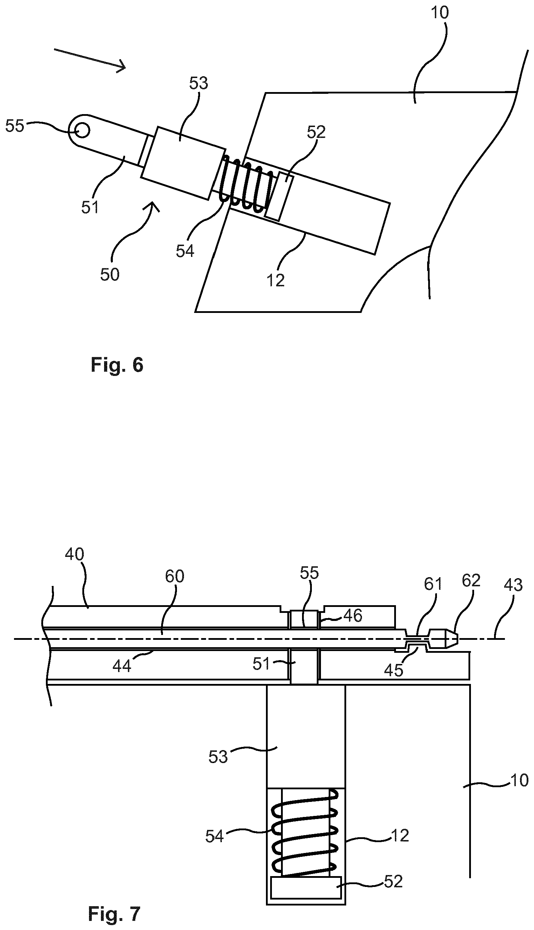

Fig. 6 zeigt eine vergrößerte Ansicht des Haltesystems bei der Montage im Pumpengehäuse 10. Dazu ist im Pumpengehäuse 10 eine zylindrische Sackbohrung 12 vorgesehen, bei der es sich jedoch auch um eine Durchgangsbohrung handeln kann. Das Haltesystem 50, bestehend aus dem Deckelhalter 51 mit dem Anschlag 52, der Gleitlagerbuchse 53 und der Druckfeder 54 kann vormontiert und als Federpaket in die Bohrung 12 eingesteckt werden. Dabei hat der Anschlag 52 einen geringeren Durchmesser als die Gleitlagerbuchse 53, so dass der Anschlag 52 in die Bohrung 12 gesteckt werden kann, bevor die Gleitlagerbuchse 53 beispielsweise mit einer Presspassung im Gehäuse 10 verankert wird. Bei einer Durchgangsbohrung kann der Anschlag 52 zusammen mit der Feder 54 auch auf der anderen Seite aus der Bohrung herausragen.

Die Außenkontur der Gleitlagerbuchse 53 kann zylindrisch ausgeformt sein, aber auch eine andere Geometrie aufweisen. Beispielsweise kommen auch quadratische, rechteckige, ovale oder polygone Querschnitte in Betracht, so dass der Querschnitt der Bohrung 12 an die Außenkontur der Gleitlagerbuchse 53 angepasst ist.

Auf der dem Anschlag 52 entgegen gesetzten Seite des Deckelhalters 51 ist eine Stifthalterung in Form einer Bohrung 55 vorgesehen. Durch diese Bohrung 55 kann ein Scharnierstift geschoben werden, welcher den Deckel 40 mit dem Haltesystem 50 verbindet. Der Scharnierstift bildet dann die Drehachse 43 des Deckels 40. Dieser Endbereich des Deckelhalters 51, in welchem die Stifthalterung 55 vorgesehen ist, kann wie der restliche Deckelhalter 51 zylindrisch ausgeformt sein. Er kann jedoch auch plattenförmig ausgeformt sein, so dass der Deckelhalter 51 in diesem Bereich einen rechteckigen Querschnitt hat. Dabei ist der Übergang zwischen dem runden und dem eckigen Querschnitt vorzugsweise so positioniert, dass er sich im Betrieb in der Nähe des Außenrands der Gleitlagerbuchse 53 befindet.

Die Anbringung des Deckels 40 am Deckelhalter 51 ist schematisch einer Aufsicht in der Fig. 7 zu entnehmen. Dabei ist das Haltesystem 50 mit der Gleitlagerbuchse 53 in der Bohrung 12 im Pumpengehäuse 10 verankert. Der Deckelhalter 51 ragt beispielsweise mit seinem plattenförmigen Abschnitt aus der Gleitlagerbuchse 53 heraus und ist in eine Bohrung 46 im Deckel 40 eingefügt. Diese Bohrung 46 verläuft quer zur Drehachse 43 des Deckels 40. Innerhalb des Deckels ist eine Scharnierbohrung 44 vorgesehen, welche entlang der Drehachse 43 verläuft. Durch diese Scharnierbohrung 44 kann ein Scharnierstift 60 geschoben werden, so dass er den Deckelhalter 51 im Bereich der Stifthalterung 55 durchdringt. Hierdurch wird in der Drehachse 43 ein Drehlager bereitgestellt, um welches der Deckel 40 schwenkbar ist, wobei eine Spielpassung realisiert wird.

Durch diesen Aufbau des Haltesystems 50 kann es mit der Gleitlagerbuchse 53 in der Bohrung 12 fixiert werden, wobei dies radial positionsunabhängig erfolgen kann. Daraufhin kann der Deckelhalter 51 so innerhalb der Gleitlagerbuchse 53 gedreht werden, dass der Deckel 40 mit der Bohrung 46 auf den plattenförmigen Abschnitt des Deckelhalters 51 gesteckt werden kann. In dieser Position kann der Scharnierstift 60 in die Scharnierbohrung 44eingeführt werden, so dass er die Stifthalterung 55 im Deckelhalter 51 durchdringt.

Um den Scharnierstift 60 innerhalb des Deckels 40 zu fixieren, weist der Scharnierstift 60 eine umlaufende Nut 61 auf. In diese Nut 61 rastet eine Erhebung 45 im Deckel 40 ein, wenn der Scharnierstift von links durch die Scharnierbohrung 44 geschoben wird. Um den Stift 60 leichter in die Bohrung 47 einführen zu können, ist er an seinem Ende vorzugsweise mit einer umlaufenden Fase 62 versehen. Durch die Verrastung mit der Nut 61 und die permanent anliegende Federkraft ist der Scharnierstift 60 gegen ein Herausgleiten aus der Bohrung 47 gesichert. Liegt der Bereich der Nut 61 frei, kann der Scharnierstift 60 jedoch durch leichtes Anheben an der Fase 62 aus der axialen Verriegelung gelöst werden.

### Bezugszeichenliste:

- 10: Pumpengehäuse
- 11: Lauffläche
- 12: Bohrung
- 13: Arterieller Luftfänger
- 14: Venöser Luftfänger
- 15: Dialysator
- 16: Dialysat
- 17: Anzeige-/Eingabeeinheit, Touchscreen
- 18: Steuer-/Regelungseinheit
- 19: Medizinisches Gerät zur extrakorporalen Blutbehandlung, Dialysegerät
- 20: Schlauchsegment, Überleitsystem
- 21: Arterielles Blut vom Patienten
- 22: Venöses Blut zum Patienten
- 30: Rotor
- 31,32: Hauptscheitel
- 40: Deckel, Pumpendeckel
- 40a: erster Gelenkabschnitt
- 41: Abroll- und Rastgeometrie
- 42: Rastfläche
- 43: Drehachse des Deckels
- 44: Scharnierbohrung
- 45: Erhebung
- 46: Bohrung
- 48,49: Abrollkurve
- 50: Haltesystem
- 51: Deckelhalter; zweiter Gelenkabschnitt
- 52: Anschlag
- 53: Gleitlagerbuchse
- 54: Feder, Druckfeder
- 55: Stifthalterung, Lagerung
- 60: Scharnierstift
- 61: Nut
- 62: Fase

## Patentansprüche

1. Medizinisches Gerät (19) zur extrakorporalen Blutbehandlung mit einer Deckelvorrichtung mit einem Deckel (40), der über zumindest ein Schwenkgelenk (40a, 51) an dem medizinischen Gerät (19) angelenkt ist, wobei
das Schwenkgelenk (40a, 51) einen, vorzugsweise am Deckel (40) angeordneten, ersten Gelenkabschnitt (40a) und einen, vorzugsweise mit dem medizinischen Gerät (19) verbindbaren, zweiten Gelenkabschnitt (51) aufweist, die zumindest zwischen einer ersten Lage, in der der Deckel (40) geöffnet ist, und einer zweiten Lage, in der der Deckel (40) zumindest einen Teil des medizinischen Geräts (19) abdeckt, zueinander um eine Schwenkachse (43) verschwenkbar und in zumindest einer Schwenklage lösbar arretierbar sind,
**dadurch gekennzeichnet, dass**
die Deckelvorrichtung zumindest ein Kontaktflächenpaar aufweist, das senkrecht zur Schwenkachse (43) federelastisch gegeneinander vorgespannt ist,
wobei zumindest eine der Kontaktflächen eine Abroll- und Rastgeometrie (41) aufweist, die so ausgebildet ist, dass der Deckel (40) in der zweiten Lage verrastet und die Schwenkbeweglichkeit des Schwenkgelenks nur unter Überwindung der federelastischen Vorspannkraft wieder freigegeben wird, und
ein relativer Abstand zwischen der Schwenkachse (43) und dem medizinischen Gerät (19) in der ersten Lage und in der zweiten Lage unterschiedlich groß ist.

2. Medizinisches Gerät (19) nach Anspruch 1, **dadurch gekennzeichnet, dass** das senkrecht zur Schwenkachse (43) federelastisch gegeneinander vorgespannte Kontaktflächenpaar durch eine auf den Deckel (40) wirkende Zugkraft vorgespannt ist.

3. Medizinisches Gerät (19) nach Anspruch 1 oder 2, **dadurch gekennzeichnet, dass**
das medizinische Gerät (19) einen Gehäuseabschnitt (10) mit wenigstens einem Haltesystem (50) aufweist, welches einen, insbesondere stift- oder zylinderförmigen, Deckelhalter (51) als zweiten Gelenkabschnitt umfasst, der federbelastet gegenüber dem medizinischen Gerät (19) bewegbar ist;
der Deckel (40) schwenkbar um die Schwenkachse (43) an dem Deckelhalter (51) gelagert ist, wobei durch den federbelasteten Deckelhalter (51) eine permanente Zugkraft auf den Deckel (40) aufbringbar ist;
die Kontaktfläche mit der Abroll- und Rastgeometrie (41) an dem am Deckel (40) angeordneten ersten Gelenkabschnitt (40a) ausgebildet ist; und
die Abroll- und Rastgeometrie (41) eine Rastfläche (42) und wenigstens eine konvexe Abrollkurve (48, 49) umfasst, wobei
die Abrollkurve (48) an einer Seite an die Rastfläche (42) angrenzt und die Rastfläche (42) in der verrasteten Lage des Deckels (40) an der anderen Kontaktfläche des Kontaktflächenpaars anliegt, während die Abrollkurve (48, 49) beim Schwenken des Deckels (40) aus der verrasteten Lage entlang der anderen Kontaktfläche des Kontaktflächenpaars abgleitet.

4. Medizinisches Gerät (19) nach einem der Ansprüche 1 bis 3, **dadurch gekennzeichnet, dass** das Haltesystem (50) eine Gleitlagerbuchse (53) umfasst, innerhalb welcher der Deckelhalter (51) axial beweglich gelagert ist, wobei die Gleitlagerbuchse (53) in einem Gehäuseabschnitt (10) des medizinischen Geräts (19) fixiert ist.

5. Medizinisches Gerät (19) nach Anspruch 4, **dadurch gekennzeichnet, dass**
der Deckelhalter (51) mit zwei gegenüber liegenden Enden aus der Gleitlagerbuchse (53) herausragt, wobei an einem Ende eine Lagerung (55) für den Deckel (40) ausgebildet ist, während der Deckelhalter (51) am anderen Ende einen Anschlag (52) aufweist, und
das Haltesystem (50) ferner eine Druckfeder (54) umfasst, welche den Deckelhalter (51) umgibt und zwischen dem Anschlag (52) und der Gleitlagerbuchse (53) gespannt ist.

6. Medizinisches Gerät (19) nach Anspruch 4, **dadurch gekennzeichnet, dass**
die andere Kontaktfläche des Kontaktflächenpaars an der Gleitlagerbuchse (53) ausgebildet ist,
die Rastfläche (42) in der verrasteten Lage des Deckels (40) an der Gleitlagerbuchse (53) anliegt, und
die wenigstens eine Abrollkurve (48, 49) beim Schwenken des Deckels (40) aus der verrasteten Lage entlang der Gleitlagerbuchse (53) abgleitet.

7. Medizinisches Gerät (19) nach einem der Ansprüche 1 bis 6, **dadurch gekennzeichnet, dass** die Abroll- und Rastgeometrie (41) zwei konvexe Abrollkurven (48, 49) aufweist, welche auf gegenüber liegenden Seiten an die Rastfläche (42) angrenzen.

8. Medizinisches Gerät (19) nach einem der Ansprüche 1 bis 7, **dadurch gekennzeichnet, dass** zwischen der wenigstens einen Abrollkurve (48; 49) und der ebenen Rastfläche (42) eine Kante ausgebildet ist.

9. Medizinisches Gerät (19) nach einem der Ansprüche 1 bis 8, **dadurch gekennzeichnet, dass** im ersten Gelenkabschnitt (40a) des Deckels (40) eine Scharnierbohrung (44) vorgesehen ist, durch welche die Drehachse (43) verläuft, und dass wenigstens eine zweite Bohrung (46) quer durch diese Scharnierbohrung (44) verläuft, wobei der Deckelhalter (51) so in die zweite Bohrung (46) eingefügt ist, dass eine Stifthalterung (55) des Deckelhalters (51) innerhalb der Scharnierbohrung (44) liegt und von einem, vorzugsweise axial innerhalb der Scharnierbohrung (44) fixierten, Scharnierstift (60) durchdrungen ist, der in die Scharnierbohrung (44) eingeschoben ist.

10. Medizinisches Gerät (19) nach einem der Ansprüche 1 bis 9, **dadurch gekennzeichnet, dass** es wenigstens eine Schlauchrollenpumpe mit einem Pumpengehäuse (10) umfasst, das eine gebogene Lauffläche (11) und einen innerhalb der Lauffläche (11) drehenden Rotor (30) aufweist, wobei ein Schlauchsegment (20) eines extrakorporalen Blutkreislaufs zwischen die Lauffläche (11) und den Rotor (30) einbringbar ist, und die Schlauchrollenpumpe eine Deckelvorrichtung gemäß einem oder mehreren der Ansprüche 1 bis 10 umfasst, wobei das Pumpengehäuse (10) in der geschlossenen Lage wenigstens teilweise durch den Deckel (40) abdeckbar ist.

## Claims

1. Medical device (19) for extracorporeal blood treatment with a lid apparatus, comprising a lid (40) which is hinged by means of at least one pivot joint (40a, 51) at the medical device (19),
wherein the pivot joint (40a, 51) comprises a first joint section (40a) which is preferably positioned at the lid (40) and a second joint section (51) which is preferably connectable to the medical device (19), wherein said joint sections are pivotable with respect to each other about a pivot axis (43) at least between a first position, in which the lid (40) is open, and a second position, in which the lid (40) covers at least a part of the medical device (19), and wherein also said joint sections are releasably lockable in at least one pivoting position,
**characterized in that**
the lid apparatus comprises at least one pair of contact surfaces which, perpendicular to the axis of rotation (43), are resiliently biased against each other, wherein at least one of the contact surfaces comprises a rolling and latching geometry (41) which is configured such that the lid (40) latches in the second position, and the pivoting movability of the pivot joint is only released when the resilient biasing force is overcome, and
a relative distance between the pivot axis (43) and the medical device (19) is different in the first position and in the second position.

2. Medical device (19) according to claim 1, **characterized in that** the pair of contact surfaces which, perpendicular to the axis of rotation (43), are resiliently biased against each other are biased by a pulling force applied to the lid (40).

3. Medical device (19) according to claim 1 or 2, **characterized in that**
the medical device (19) comprises a housing section (10) having at least one retention system (50) including a lid support (51), in particular a pin or cylindrically shaped lid support, as the second joint section, which is movable with respect to the medical device (19) under load of a spring;
the lid (40) is pivotably mounted about the pivot axis (43) at the lid support (51), wherein a constant pulling force can be applied to the lid (40) by the spring loaded lid support (51);
the contact surface comprising the rolling and latching geometry (41) is configured at the first joint section (40a) positioned at the lid (40); and
the rolling and latching geometry (41) includes a latching surface (42) and at least one convex rolling curve (48, 49), wherein
the rolling curve (48) borders on the latching surface (42) on one side, and the latching surface (42), when the lid (40) is in the latched position, bears on the other contact surface of the pair of contact surfaces, while, upon pivoting of the lid (40), the rolling curve (48, 49) slides from the latched position along the other contact surface of the pair of contact surfaces.

4. Medical device (19) according to one of claims 1 to 3, **characterized in that**, the retention system (50) includes a plain bearing bush (53) within which the lid support (51) is axially movably mounted, wherein the plain bearing bush (53) is fixed in a housing section (10) of the medical device (19).

5. Medical device (19) according to claim 4, **characterized in that**
the lid support (51) protrudes from the plain bearing bush (53) with two ends lying opposite each other, wherein a bearing (55) for the lid (40) is configured at one end, while at the other end the lid support (51) comprises a stop (52), and
the retention system (50) further includes a compression spring (54) which surrounds the lid support (51) and is biased between the stop (52) and the plain bearing bush (53).

6. Medical device (19) according to claim 4, **characterized in that**
the other contact surface of the pair of contact surfaces is configured at the plain bearing bush (53),
the latching surface (42) bears on the plain bearing bush (53) when the lid (40) is in the latched position, and
the at least one rolling curve (48, 49), upon pivoting of the lid (40), slides from the latched position along the plain bearing bush (53).

7. Medical device (19) according to one of claims 1 to 6, **characterized in that** the rolling and latching geometry (41) comprises two convex rolling curves (48, 49) which border the latching surface (42) on opposite lying sides.

8. Medical device (19) according to one of claims 1 to 7, **characterized in that** an edge is configured between the at least one rolling curve (48, 49) and the level latching surface (42).

9. Medical device (19) according to one of claims 1 to 8, **characterized in that**
a hinge hole (44) is provided in the first joint section (40a) of the lid (40), through which the axis of rotation (43) passes, and that at least one second hole (46) passes transversely through said hinge hole (44), wherein
the lid support (51) is inserted in the second hole (46) such that a pin support (55) of the lid support (51) lies inside the hinge hole (44) and is penetrated by a hinge pin (60) which is preferably fixed axially inside the hinge hole (44), wherein the hinge pin (60) is inserted in the hinge hole (44).

10. Medical device (19) according to one of the claims 1 to 9, **characterized by comprising**
at least one peristaltic pump having a pump housing (10) which comprises a curved running surface (11) and a rotor (30) that rotates inside the running surface (11), wherein
a tube segment (20) of an extracorporeal blood circuit is introducible between the running surface (11) and the rotor (30), and
the peristaltic pump includes a lid apparatus according to one or more of claims 1 to 10, wherein the pump housing (10) in the closed position is at least partially coverable by the lid (40).

## Revendications

1. Appareil médical (19) pour un traitement sanguin extracorporel comprenant un dispositif de recouvrement avec un couvercle (40) qui est articulé sur l'appareil médical (19) via au moins une articulation pivotante (40a, 51) ; dans lequel
l'articulation pivotante (40a, 51) présente une première section d'articulation (40a) agencée de préférence sur le couvercle (40) et une seconde section d'articulation (51) qui peut être raccordée de préférence à l'appareil médical (19), lesquelles sections peuvent pivoter l'une par rapport à l'autre autour d'un axe de pivotement (43) au moins entre une première position, dans laquelle le couvercle (40) est ouvert, et une seconde position dans laquelle le couvercle (40) recouvre au moins une partie de l'appareil médical (19) et peuvent être bloqués de manière amovible au moins dans une position de pivotement,
**caractérisé en ce que**
le dispositif de recouvrement présente au moins une paire de surfaces de contact qui sont précontraintes perpendiculairement à l'axe de pivotement (43) de manière élastique l'une vis-à-vis de l'autre,
dans lequel au moins l'une des surfaces de contact présente une géométrie de déroulement et d'arrêt (41) qui est conçue de sorte que le couvercle (40) soit bloqué dans la seconde position et que la capacité de déplacement pivotante de l'articulation pivotante ne soit à nouveau libérée qu'en surmontant la force de précontrainte élastique et
une distance relative entre l'axe de pivotement (43) et l'appareil médical (19) a une grandeur différente dans la première position et dans la seconde position.

2. Appareil médical (19) selon la revendication 1, **caractérisé en ce que** la paire de surfaces de contact précontrainte perpendiculairement à l'axe de pivotement (43) de manière élastique l'une vis-à-vis de l'autre est précontrainte par une force de traction agissant sur le couvercle (40).

3. Appareil médical (19) selon la revendication 1 ou 2, **caractérisé en ce que**
l'appareil médical (19) présente une section de boîtier (10) avec au moins un système de support (50) qui comprend un support de couvercle (51) en particulier en forme de broche ou de cylindre comme seconde section d'articulation qui peut être déplacée sous l'effet d'un ressort vis-à-vis de l'appareil médical (19) ;
le couvercle (40) est monté à pivotement autour de l'axe de pivotement (43) sur le support de couvercle (51), dans lequel une force de traction permanente peut être appliquée au couvercle (40) par le support de couvercle (51) sollicité par un ressort ;
la surface de contact avec la géométrie de déroulement et d'arrêt (41) est formée sur la première section d'articulation (40a) agencée sur le couvercle (40) ; et
la géométrie de déroulement et d'arrêt (41) comprend une surface d'arrêt (42) et au moins une courbe de déroulement convexe (48, 49), dans lequel
la courbe de déroulement (48) est limitrophe d'un côté de la surface d'arrêt (42) et la surface d'arrêt (42) s'appuie, dans la position enclenchée du couvercle (40) sur l'autre surface de contact de la paire de surfaces de contact, tandis que la courbe de déroulement (48, 49) coulisse, lors du pivotement du couvercle (40) depuis la position enclenchée le long de l'autre surface de contact de la paire de surfaces de contact.

4. Appareil médical (19) selon l'une quelconque des revendications 1 à 3, **caractérisé en ce que** le système de support (50) comprend un coussinet lisse (53) à l'intérieur duquel le support de couvercle (51) est monté de manière à pouvoir se déplacer axialement, dans lequel le coussinet lisse (53) est fixé dans une section de boîtier (10) de l'appareil médical (19).

5. Appareil médical (19) selon la revendication 4, **caractérisé en ce que**
le support de couvercle (51) dépasse du coussinet lisse (53) par deux extrémités disposées l'une en face de l'autre, dans lequel est formé à une extrémité un logement (55) pour le couvercle (40), tandis que le support de couvercle (51) présente une butée (52) à l'autre extrémité et
le système de support (50) comprend en outre un ressort de compression (54) qui entoure le support de couvercle (51) et est tendu entre la butée (52) et le coussinet lisse (53).

6. Appareil médical (19) selon la revendication 4, **caractérisé en ce que**
l'autre surface de contact de la paire de surfaces de contact est formée sur le coussinet lisse (53),
la surface d'arrêt (42) s'appuie sur le coussinet lisse (53) dans la position enclenchée du couvercle (40) et
la au moins une courbe de déroulement (48, 49) coulisse le long du coussinet lisse (53) lors du pivotement du couvercle (40) depuis la position enclenchée.

7. Appareil médical (19) selon l'une quelconque des revendications 1 à 6, **caractérisé en ce que** la géométrie de déroulement et d'arrêt (41) présente deux courbes de déroulement convexes (48, 49) qui sont contiguës sur les côtés en regard l'un de l'autre de la surface d'arrêt (42).

8. Appareil médical (19) selon l'une quelconque des revendications 1 à 7, **caractérisé en ce qu'**une arête est formée entre la au moins une courbe de déroulement (48, 49) et la surface d'arrêt plane (42).

9. Appareil médical (19) selon l'une quelconque des revendications 1 à 8, **caractérisé en ce qu'**il est prévu dans la première section d'articulation (40a) du couvercle (40) un alésage de charnière (44) à travers lequel l'axe de rotation (43) s'étend et au moins un second alésage (46) s'étend transversalement à cet alésage de charnière (44), dans lequel le support de couvercle (51) est ajusté dans le second alésage (46) de sorte qu'une fixation à broche (55) du support de couvercle (51) se trouve à l'intérieur de l'alésage de charnière (44) et soit traversée par une broche de charnière (60) fixée de préférence axialement dans l'alésage de charnière (44), laquelle broche est inséré dans l'alésage de charnière (44).

10. Appareil médical (19) selon l'une quelconque des revendications 1 à 9, **caractérisé en ce qu'**il comprend au moins une pompe à rouleau de tuyau souple avec un boîtier de pompe (10) qui présente une surface de roulement arquée (11) et un rotor (30) tournant à l'intérieur de la surface de roulement (11), dans lequel un segment de tuyau souple (20) d'un circuit sanguin extracorporel peut être inséré entre la surface de roulement (11) et le rotor (30) et la pompe à rouleau de tuyau souple comprend un dispositif de recouvrement selon une ou plusieurs des revendications 1 à 10, dans lequel le boîtier de pompe (10) peut être recouvert au moins en partie par le couvercle (40) en position fermée.
